# EUROPÄISCHE PATENTSCHRIFT

(11) **EP 1 307 173 B1**
(45) Veröffentlichungstag und Bekanntmachung des Hinweises auf die Patenterteilung: **15.10.2008**
(21) Anmeldenummer: 01965176.9
(22) Anmeldetag: 06.08.2001
(51) Int. Cl.: A61K 6/093, A61K 6/02

(54) **HAFTENDER FLUORIDLACK**
ADHESIVE FLUORIDE VARNISH
VERNIS ADHESIF A BASE DE FLUORURES

(30) Priorität: 07.08.2000 DE 10039298; 17.08.2000 DE 10040716
(43) Veröffentlichungstag der Anmeldung: 07.05.2003
(73) Patentinhaber: S & C Polymer Silicon- und Composite-Spezialitäten GmbH, 25335 Elmshorn (DE)
(72) Erfinder: ENGELBRECHT, Jürgen, 22607 Hamburg (DE)
(74) Vertreter: Forstmeyer, Dietmar
(86) Internationale Anmeldenummer: PCT/EP2001/009072
(87) Internationale Veröffentlichungsnummer: WO 2002/011681

(56) Entgegenhaltungen:
- EP-A- 1 022 012
- WO-A-92/16183
- DE-A- 19 934 117
- DATABASE WPI Section Ch, Week 199941 Derwent Publications Ltd., London, GB; Class A26, AN 1999-488737 XP002185253 & JP 11 209213 A (KURARAY CO LTD), 3 August 1999 (1999-08-03)

## Beschreibung

Die Erfindung betrifft Mischungen, die Polymere mit Hydrogensiloxangruppen und Fluoride enthalten, sowie ihre Verwendung als dentaler Versiegelungs- und Fluoridierungslack, Desensibilisierungsmittel sowie als Schutzlack für Glasionomerzementfüllungen.

Schutzlacke für Zähne werden seit fast drei Jahrzehnten erfolgreich angewandt. Da sind zum einen die Fissurenversiegelungslacke basierend auf mit Licht polymerisierbaren (1-Komponen-ten) oder selbstpolymerisierenden (2-Komponenten) Methacrylat-harzen. Ihre Anwendung bedingt ein vorheriges Anätzen der Schmelzoberfläche mit Phosphorsäure. Die Haftung auf dem Zahnschmelz erfolgt micromechanisch, der Schutzlack versiegelt Microfissuren über Jahre. In einigen Formulierungen sind den Lacken Fluoridsalze, fluorabgebende Glaspulver oder organische Fluoridverbindungen zugemischt. Klassische Beispiele sind Delton (Dentsply, USA), Visio-Seal (Espe, Deutschland).

Zum anderen gibt es Fluoridlacke für Zähne, Polymerlösungen, die Fluoride enthalten, und die nach dem Abdampfen des Lösungsmittels einen nagellackähnlichen farblosen Schutzfilm auf der Zahnoberfläche hinterlassen. Filme dieser Art verbleiben nur kurzfristig auf der Oberfläche des Zahnes und dienen lediglich Depot für das zu applizierende Fluorid. Beispiele dieser Gruppe von Fluoridlacken sind Produkte wie Bifluorid (Voco, Deutschland), Duraphat (Colgate, Deutschland).

Eine weitere Entwicklung der Zahnlacke stellen Addukte aus Siliconen und säurehaltigen Monomeren (K.G. Bohlig et al., Abstract 1036, J. Dent. Research, Vol. 79, Spec. Issue 2000) dar. Hier werden Polymere, die Bruchstücke aus Silicon mit Si-OH- und -COOH -Gruppen enhalten, als Lösung auf die Zahnoberfläche aufgetragen. Ihre doppelte Funktionalität macht es möglich, sowohl auf dem Zahnschmelz ohne Anätzung zu haften (-COOH) als auch untereinander bei Anwesenheit potentieller Kondensationskatalysatoren und Vernetzer zu vernetzen. (SiOSiOH + Si(OR)₄ + HOSiOSi ----> SiOSiO-OSiO-SiOSi). Lacke dieser Art sind hydrophober als bisherige und von daher weniger leicht auswaschbar, haften und sind, wenn vernetzt, recht dauerhaft.

In der PCT/EP 0001042 und EP 0 632 060 A1 werden Haftvermittler aus Copolymeren enthaltend Hydrogensiloxangruppen sowie Lösungen daraus beschrieben. Haftvermittler der PCT/EP 0001042 haften unerwartet auch an Zahnsubstanz.

Die Druckschrift EP 1022012 betrifft Dentalmaterialien auf der Basis von polymerisationsfähigen, Methacrylat-modifizierten Polysiloxanen. Diese Druckschrift beschreibt Dentalmaterialien auf Basis von Polysiloxanen, die sich in organischanorganische Verbundmaterialien kovalent einbauen lassen und keine Spiro- oder Thioethergruppen enthalten.

Aufgabe der vorliegenden Erfindung war es, einen neutralen Lack zu finden, der in der Lage ist, einen haftenden potentiell vernetzenden Zahnüberzug zu bilden und der als Träger für Fluoridierungsmittel dienen kann.

Erfindungsgemäß wurde die Aufgabe gelöst durch Mischungen, die
a) Polymere mit Hydrogensiloxangruppen,
b) Fluoride,
c) gegebenenfalls einen Platinkatalysator, und
d) gegebenenfalls Vinylverbindungen
enthalten.

Die erfindungsgemäßen Mischungen enthalten als wichtige lackbildende und auf Zahnsubstanz haftende Substanz Polymere mit Hydrogensiloxangruppen. Das Grundgerüst dieser Polymeren, können z.B. Copolymere aus Methylmethacrylat und Allylmethacrylat sein, denen Hydrogensiloxan aufgepropft ist. Es können auch Copolymere sein, wie sie ausführlich in der EP 0 632 060 A1 beschrieben sind oder auch, besonders bevorzugt, Copolymere, die durch direkte Polymerisation von Hydrogensiloxanen gewonnen sind wie in der beigefügten PCT/EP 0001042 und in der Japanischen Offenlegungsschrift (A) Nr. HEI 10-25322, offengelegt am 27.01.1998, beschrieben sind. Auf die drei vorgenannten Druckschriften wird bezüglich der (Co-)Polymere und deren Herstellung ausdrücklich Bezug genommen. Die SiH-Gruppen der Hydrogensiloxaneinheiten vermögen offenbar mit den POH-Gruppen des Apatiths der Zahnsubstanz zu reagieren, so daß ohne vorherige mechanische Aufrauhung (Ätzung) eine Bindung des Lackes zur Zahnsubstanz auf chemischen Wege zustande kommt.

Die eingesetzten Fluoride können die gleichen sein wie sie in den handelsüblichen Fluoridlacken enthalten sind. Vielfach eingesetzte Fluoride sind anorganische oder organische Fluoride, wie Natriumfluorid, Kaliumfluorid, Calziumfluorid, Strontiumfluorid, Zinkfluorid, Zinnfluorid, Natrium- oder Kaliumhexafluorphospat, Natrium-oder Kalium-hexafluo-raluminat, Ammoniumfluorid oder organische Fluoride wie z.B. Aminfluoride wie Cetylaminhydrofluorid, Bis(hydroxyethyl)-aminopropyl-N-hydroxyethyloctadecylamindihydrofluorid.

Erfindungsgemäß können feste Fluoride feinstmöglich, z.B bis zu einer mittleren Körngröße zwischen 1 bis 10 Micron gemahlen oder in einem der nachstehend erwähnten Lösungsmitteln gelöst werden.

Flüssige Fluoride können als solche oder in einem der nachstehend erwähnten Lösungsmitteln gelöst eingesetzt werden.

Die erfindungsgemäßen Mischungen weisen die Polymere mit Hydrogensiloxangruppen und die Fluoride vorzugsweise in einem Gewichtsverhältnis von 100 : 1 bis zu 10 : 5, besonders bevorzugt in einem Gewichtsverhältnis von 20 : 1 bis zu 10 : 4 auf.

Zusätzlich können sie bis zu 10, vorzugsweise bis zu 1 Gewichtsteil einer Vinylverbindung, bis zu 500 ppm, vorzugsweise bis zu 50 ppm Platinkatalysator und bis zu 80, vorzugsweise bis zu 20 Gewichtsteile Polymer(e) ohne Hydrogensiloxangruppen enthalten.

Die SiH-Gruppen der Hydrogensiloxaneinheiten der erfindungsgemäß eingesetzten Polymere zeigen eine Tendenz im feuchten Milieu zu hydrolysieren (SiH ---> SiOH) und neigen dann dazu, unter Abspaltung von Wasserstoff zu vernetzen (SiH + SiOH ---> SiOSi + H₂). Im Mundmilieu spielt sich diese Abspaltung von Wasserstoff nach Applikation des Zahnlackes ab, führt somit zu einer nachträglichen Vernetzung. Ein Katalysator ist somit nicht unbedingt nötig.

Sollte es gewünscht sein, eine schnelle Vernetzung der Lackpolymere, besonders in Gegenwart von Vinyl- oder anderen doppelbindungshaltigen Verbindungen zu erreichen, so kann es nützlich sein, gegebenenfalls einen Katalysator zuzugeben, der die Reaktionen SiH + SiOH oder SiH +-CR=CR2 zu katalysieren vermag. Bekannterweise sind dieses Verbindungen des Platins oder Palladiums sowie Legierungen von diesen. Besonders geeignet sind Platin(0)-Verbindungen, die vom Platinhexachloroplatinat ausgehend hergestellt sind und welche üblich sind in Systemen additionsvernetzender Vinylpolysiloxane / Hydrogenpolysiloxane.
Beispiele derartiger Katalysatoren sind:
Ptₓ (Divinyltetramethyldisiloxan)y des Platin(0).

Gegebenenfalls zugesetzte Vinylverbindungen sollten bevorzugt Divinylverbindungen sein. Aber auch Verbindungen mit mehr als zwei Vinylgruppen können sinnvoll sein, so z.B., wenn ein hoher Vernetzungsgrad erreicht werden soll. Bevorzugte DivinylVerbindungen sind Divinylether wie Divinylether von Triethylenglycol oder Cyclohexandiol oder Divinylpolysiloxane wie sie in additionsvernetzenden Siliconen verwendet werden oder auch Divinyltetramethyldisiloxan.

Erfindungsgemäße Mischungen können zusätzlich auch Polymere enthalten, die keine SiH-Gruppen aufzeigen. Beispiele dafür sind Polymethacrylate, Polycarbonate, Polyester und ähnliche Polymere sofern sie in der Mischung löslich und mit dem System verträglich sind.

Ebenfalls können erfindungsgemäße Mischungen Lösungsmittel, vorzugsweise leicht flüchtige Lösungsmittel, enthalten, die es ermöglichen, den Lack als sehr dünnen Film aufzutragen. Als Lösungsmittel eignen sich besonders leichtflüchtige inerte Lösungsmittel wie halogenierte oder nichthalogenierte aliphatische oder aromatische Kohlenwasserstoffe, Ether, Ketone, Ester oder cyclische Siloxane. Insbesondere werden physiologisch unbedenkliche Lösungsmittel eingesetzt und besonders bevorzugt Lösungsmittel, in denen sich die erfindungsgemäß eingesetzten löslichen Fluoride gut lösen.

Zur Stabilisierung von nichtlöslichen Fluoridpulverteilchen können den erfindungsgemäßen Mischungen Hilfsstoffe zugegeben werden, die vorzeitigen Entmischungen entgegenwirken können. Solche Stoffe können z.B. pyrogene Kieselsäuren oder modifizierte Cellulosen sein. Auch Zusätze von Agentien, die ein Zusammenbacken abgesetzter Teilchen verhindern, können sinnvoll und angezeigt sein, wenn die Mischungen über längere Zeit gelagert werden und durch leichtes Aufschütteln wieder homogenisiert bar sein sollen. Auch können Verbindungen oder Mittel zugesetzt werden, die biostatische, biozide oder pharmazeutische Wirkungen wie z.B. antibacterielle oder entzündungshemmende Wirkungen aufzeigen. Biozide Verbindungen oder Mittel töten Bakterien, Viren und/oder Pilze, während biostatische Verbindungen oder Mittel das Wachstum von Bakterien, Viren und/oder Pilzen hemmen.

Gemäß einer weiteren Ausführungsform können die erfindungsgemäßen Mischungen zusätzlich von a) verschiedene Hydrogensiloxane enthalten. Derartige Hydrogensiloxane sind vorzugsweise Polysiloxane mit mindestens zwei, vorzugsweise drei SiH-Gruppen.

Erfindungsgemäße Mischungen eignen sich hervorragend als haftende Zahnlacke zur Fissurenversiegelung ohne Ätzung durch Phosphorsäure. Durch die fehlende Ätzung kann vermieden werden, daß gesunde Schmelzsubstanz über den Bereich der Versiegelung hinaus geschädigt wird, andererseits liegen keine ungeätzten nicht bindenden Flächen unter der Versiegelung vor. Ein großer Vorteil liegt auch darin, daß Versiegelungen mit erfindungsgemäßen Lacken ohne weiteres ergänzt werden können, was mit Fissurenversiegelungsmaterialien auf Methacrylatbasis nicht so innig möglich ist. Ein weiterer Vorteil ist, daß erfindungsgemäße Versiegelungen in extrem dünner Schicht erfolgen können, was bei Methacrylatversiegelungen wegen der 50 - 100 Micron Inhibitionsschicht durch Anwesenheit von Luftsauerstoff nicht möglich ist.

Auch als haftender Lack zur Fluoridierung des Zahnschmelzes sind die erfindungsgemäßen Lacke den bisherigen Fluoridierungslacken überlegen: Sie haften chemisch, also wesentlich besser, sind hydrophober, mehrere Monate selbst auf belasteten Flächen haltbar und können jederzeit leicht wieder ergänzt werden.

Auch zur Desensibilisierung von Zahnhälsen sind erfindungsgemäße Mischungen geeignet. Aufgetragen verschließen sie nach Verdampfen des Lösungsmittels schnell offene Dentinkanälchen und verhindern so die schmerzhaften Beschwerden.

Es hat sich unvorhergesehen auch gezeigt, daß sich erfindungsgemäße Lacke hervorragend als Schutzlacke für Zemente wie Dentalzemente eignen. So muß z.B. die Oberfläche frisch angemischter und als Füllung applizierter Glasionomerzemente mit einem Schutzlack versehen werden. Schutzlacke dieser Art sind bisher Lösungen von Polymeren wie Polymethylmethacrylate, Polyester, Polycarbonate o.ä.. Diese Lacke verbleiben nur wenige Stunden oder Tage auf der Oberfläche der frischen Zementfüllung; sie haften nur mechanisch und sind schnell weggewaschen. Sie erfüllen zwar weitgehend ihre Aufgabe, da die Glasionomerzemente hauptsächlich in den ersten Minuten bis Stunden geschützt werden müssen, bis eben der Erhärtungsmechanismus zu weniger löslichen, raumvernetzten Strukturen geführt hat. Die erfindungsgemäßen Lacke haben dennoch den Vorteil, daß sie zum einen hydrophober sind und so ein Weglösen darunterliegender Schichten des Zementes besser verhindern, zum anderen schont und ergänzt der Fluoridgehalt der erfindungsgemäßen Lacke das Fluorreservoir des Glasionomerzementes und verlängert damit dessen Wirksamkeit. Auch können spätere Applikation erfindungsgemäßer Lacke auf Glasionomerfüllungen zu einem "Wiederaufladen" von Fluorid in der Glasionomerfüllung führen, was bekanntermaßen mit Fluoridapplikationen möglich ist und was durch die lange Gegenwart des erfindungsgemäßen Lackes auf der Glasionomeroberfläche intensiv und nachhaltig stattfinden kann.

Alle folgenden Mengenangaben sind, soweit nicht anders angegeben, auf das Gewicht bezogen.

### Beispiele

### Beispiel 1

### Fissurenversiegelungsmaterial

Ein Hydrogensiloxanhaltiges Copolymer wurde nach Beispiel 1 der PCT/EP 00/01042 hergestellt. Eine erfindungsgemäße Mischung wird frisch hergestellt aus 10 Teilen des Polymers, 2 Teilen feinstgemahlenem Natriumfluorid, 0,2 Teilen Ethylcellulose, 0,05 Teilen Titandioxid und 57 Teilen Ethylacetat. Nach kurzem aber intensivem mechanischen Reinigen mit einer rotierenden Bürste mit Polierpaste, Reinigen mit Ethanol mit anschließendem Spülen mit Wasser, Trocknen mit ölfreier Luft, werden die Fissuren der occlusalen Fläche eines Milchmolarenzahnes zweimal mit Hilfe eines Dentalpinsels mit der Lösung dünn bestrichen. Nach 8 Wochen ist die Versiegelung noch sichtbar.

### Beispiel 2

### Antiplaque-Fluörid-Lack

Eine erfindungsgemäße Mischung wird frisch hergestellt aus 10 Teilen des Polymers aus Beispiel 1, 0,2 Teilen feinstgemahlenem Zinkfluorid, 0,1 Teilen Aerosil 810 (Degussa), 89 Teilen Ethylacetat. Nach kurzem aber intensivem mechanischen Reinigen mit einer rotierenden Bürste mit Polierpaste, Reinigen mit Ethanol mit anschließendem Spülen mit Wasser, Trocknen mit ölfreier Luft, wird die labiale Fläche eines Frontzahnes einmal mit Hilfe eines Dentalpinsels mit der Lösung dünn bestrichen. Nach 8 Wochen wird mit Hilfe eines Plaque- Anfärbetests auf Plaque geprüft. Auf der erfindungsgemäß behandelten Oberfläche kann kein Plaque erkannt werden, während alle anderen Zähne mehr oder minder ausgeprägte Plaqueerscheinungen aufweisen.

### Beispiel 3

### Desensitizer

Die Lösung von Beispiel 2 wird nach entsprechender Vorreinigung auf einen sensiblen Zahnhals gegeben. Nach kurzer Zeit ist die Empfindlichkeit verschwunden. Nach 8 Wochen ist die dünne Fluoridlackschicht noch erkennbar.

### Beispiel 4

### Zementlack

Ein Hydrogensiloxanhaltiges Copolymer wurde nach Beispiel 1 der PCT/EP 00/01042 hergestellt. Eine erfindungsgemäße Mischung wird frisch hergestellt aus 12 Teilen des Polymers (siehe Beispiel 1), 1 Teil feinstgemahlenem Natriumfluorid, 0,1 Teilen Ethylcellulose und 90 Teilen Methylethylketon. Ein Probekörper wird aus frisch angemischtem Glasionomerzement (Aqua Ionofil Plus, Voco, Deutschland) hergestellt, die Oberfläche mit dem erfindungsgemäßen Lack bestrichen und die Probe sofort in Wasser gegeben. Die gleiche Prozedur wird wiederholt, dieses Mal jedoch ohne die Oberfläche erfindungsgemäß zu behandeln. Nach einer Woche werden die Proben dem Wasser entnommen. Die Oberfläche des erfindungsgemäß behandelten Probekörpers ist deutlich glatter und kratzfester als der nichtbehandelte Probekörper.

## Patentansprüche

1. Mischungen, die
a) Polymere mit Hydrogensiloxangruppen,
b) Fluoride,
c) gegebenenfalls einen Platinkatalysator, und
d) gegebenenfalls Vinylverbindungen
enthalten.

2. Mischungen nach Anspruch 1, **dadurch gekennzeichnet, daß** die Polymere mit Hydrogensiloxangruppen Copolymere von ungesättigten Verbindungen sind, denen SiH-Gruppen-haltige Siloxane aufgepfropft sind.

3. Mischungen nach Anspruch 1, **dadurch gekennzeichnet, daß** die Polymere mit Hydrogensiloxangruppen Copolymere von SiH-Gruppen-haltigen Siloxanen mit ungesättigten Verbindungen sind.

4. Mischungen nach einem der Ansprüche 1 - 3, **dadurch gekennzeichnet, daß** die Fluoride Natrium-, Calzium- oder Zinnfluoride, Hexafluorphosphate oder Aminfluoride sind.

5. Mischungen nach einem der Ansprüche 1 - 4, **dadurch gekennzeichnet, daß** der Platinkatalysator eine Verbindung des Platin (0) ist.

6. Mischungen nach einem der Ansprüche 1 - 5, **dadurch gekennzeichnet, daß** die Vinylverbindungen Vinylsiloxan- oder Vinylether-Gruppen aufweisen.

7. Mischungen nach einem der Ansprüche 1 - 6, **dadurch gekennzeichnet, daß** sie zusätzlich Polymere ohne Hydrogensiloxangruppen enthalten.

8. Mischungen nach einem der Ansprüche 1 - 7, **dadurch gekennzeichnet, daß** leicht flüchtige Lösungsmittel enthalten sind.

9. Mischungen nach einem der Ansprüche 1 - 8, **dadurch gekennzeichnet, daß** sie zusätzlich Verbindungen oder Mittel mit biostatischer, biozider oder anderer pharmazeutischer Wirkung enthalten.

10. Mischungen nach einem der Ansprüche 1 - 9, **dadurch gekennzeichnet, daß** sie zusätzlich von a) verschiedene Hydrogen-siloxane enthalten.

11. Versiegelungslack für Zähne, **dadurch gekennzeichnet, dass** er eine Mischung nach einem der Ansprüche 1 - 10 enthält.

12. Desensibilisierer für Zahnhälse, **dadurch gekennzeichnet, dass** er eine Mischung nach einem der Ansprüche 1 - 10 enthält.

13. Schutzlack für Zemente, **dadurch gekennzeichnet, dass** er eine Mischung nach einem der Ansprüche 1 - 10 enthält.

14. Schutzlack für Dentalzemente, **dadurch gekennzeichnet, dass** er eine Mischung nach einem der Ansprüche 1 - 10 enthält.

## Claims

1. Mixtures containing
a) polymers having hydrogen-siloxane groups,
b) fluorides,
c) optionally a platinum catalyst, and
d) optionally vinyl compounds.

2. The mixtures according to Claim 1, **characterized in that** the polymers having hydrogen-siloxane groups are copolymers of unsaturated compounds onto which SiH groups-containing siloxanes have been grafted.

3. The mixtures according to Claim 1, **characterized in that** the polymers having hydrogen-siloxane groups are copolymers of SiH groups-containing siloxanes with unsaturated compounds.

4. The mixtures according to any one of Claims 1 to 3, **characterized in that** the fluorides are sodium fluorides, calcium fluorides or tin fluorides, hexafluorophosphates or amine fluorides.

5. The mixtures according to any one of Claims 1 to 4, **characterized in that** the platinum catalyst is a compound of platinum(0).

6. The mixtures according to any one of Claims 1 to 5, **characterized in that** the vinyl compounds have vinylsiloxane groups or vinyl ether groups.

7. The mixtures according to any one of Claims 1 to 6, **characterized in that** they additionally contain polymers without hydrogen-siloxane groups.

8. The mixtures according to any one of Claims 1 to 7, **characterized in that** they contain readily volatile solvents.

9. The mixtures according to any one of Claims 1 to 8, **characterized in that** they additionally contain compounds or agents having a biostatic, biocidal or other pharmaceutical effect.

10. The mixtures according to any one of Claims 1 to 9, **characterized in that** they additionally contain hydrogen-siloxanes other than those according to a).

11. A sealing varnish for teeth, **characterized in that** it contains a mixture according to any one of Claims 1 to 10.

12. A desensitizer for the necks of teeth, **characterized in that** it contains a mixture according to any one of Claims 1 to 10.

13. A protective varnish for cement, **characterized in that** it contains a mixture according to any one of Claims 1 to 10.

14. A protective varnish for dental cement, **characterized in that** it contains a mixture according to any one of Claims 1 to 10.

## Revendications

1. Mélanges contenant
a) des polymères incluant des groupes hydrogène-siloxane,
b) des fluorures,
c) éventuellement un catalyseur au platine et
d) éventuellement des composés de vinyle.

2. Mélanges selon la revendication 1, **caractérisés en ce que** les polymères incluant des groupes hydrogène-siloxane sont des copolymères de composés insaturés, auxquels sont greffés des siloxanes contenant des groupes SiH.

3. Mélanges selon la revendication 1, **caractérisés en ce que** les polymères incluant des groupes hydrogène-siloxane sont des copolymères de siloxanes contenant des groupes SiH à composés insaturés.

4. Mélanges selon l'une quelconque des revendications 1 à 3, **caractérisés en ce que** les fluorures sont des fluorures de sodium, de calcium ou d'étain, des hexafluorophosphates ou des amine-fluorures.

5. Mélanges selon l'une quelconque des revendications 1 à 4, **caractérisés en ce que** le catalyseur au platine est un composé de platine (0).

6. Mélanges selon l'une quelconque des revendications 1 à 5, **caractérisés en ce que** les composés de vinyle présentent des groupes vinyle-siloxane ou vinyléther.

7. Mélanges selon l'une quelconque des revendications 1 à 6, **caractérisés en ce qu'**ils contiennent en plus des polymères sans groupes hydrogène-siloxane.

8. Mélanges selon l'une quelconque des revendications 1 à 7, **caractérisés en ce qu'**ils contiennent des solvants facilement volatils.

9. Mélanges selon l'une quelconque des revendications 1 à 8, **caractérisés en ce qu'**ils contiennent en outre des composés ou des agents à effet biostatique, biocide ou à autre effet pharmaceutique.

10. Mélanges selon l'une quelconque des revendications 1 à 9, **caractérisés en ce qu'**ils contiennent en outre des groupes hydrogène-siloxane différents de a).

11. Vernis adhésif pour dents, **caractérisé en ce qu'**il contient un mélange selon l'une quelconque des revendications 1 à 10.

12. Désensibilisateur pour collets de dents, **caractérisé en ce qu'**il contient un mélange selon l'une quelconque des revendications 1 à 10.

13. Vernis protecteur pour ciments, **caractérisé en ce qu'**il contient un mélange selon l'une quelconque des revendications 1 à 10.

14. Vernis protecteur pour ciments dentaires, **caractérisé en ce qu'**il contient un mélange selon l'une quelconque des revendications 1 à 10.
